# EUROPEAN PATENT SPECIFICATION

(11) **EP 2 635 275 B1**
(45) Date of publication and mention of the grant of the patent: **06.08.2014**
(21) Application number: 11797264.6
(22) Date of filing: 02.11.2011
(51) Int. Cl.: A61K 31/4196, A61K 31/425, C07D 249/10, C07D 401/06, C07D 413/12, C07D 249/16, C07D 487/04, A61P 9/10, A61P 25/28

(54) **GPR17-MODULATING COMPOUNDS, DIAGNOSTIC AND THERAPEUTIC USES THEREOF**
GPR17-MODULIERENDE VERBINDUNGEN SOWIE IHRE DIAGNOSTISCHE UND THERAPEUTISCHE VERWENDUNG
COMPOSÉS MODULATEURS DE GPR17, LEURS UTILISATIONS EN DIAGNOSTIC ET EN THÉRAPIE

(30) Priority: 03.11.2010 IT MI20102037
(43) Date of publication of application: 11.09.2013
(73) Proprietor: Centro Cardiologico Monzino SpA, 20138 Milano (IT)
(72) Inventor: ABBRACCHIO, Mariapia, 20146 Milano (IT); EBERINI, Ivano, 20092 Cinisello Balsamo (mi) (IT); PARRAVICINI, Chiara, 20100 Milano (IT); MARTINI, Claudia, 56126 Pisa (IT); TRINCAVELLI, Maria Letizia, 56126 Pisa (IT); DANIELE, Simona, 56126 Pisa (IT)
(74) Representative: Banfi, Paolo
(86) International application number: PCT/IB2011/054865
(87) International publication number: WO 2012/059869

(56) References cited:
- WO-A2-2006/045476
- PARRAVICINI CHIARA ET AL: "GPR17: Molecular modeling and dynamics studies of the 3-D structure and purinergic ligand binding features in comparison with P2Y receptors", BMC BIOINFORMATICS, BIOMED CENTRAL, LONDON, GB, vol. 9, no. 1, 4 June 2008 (2008-06-04), page 263, XP021031843, ISSN: 1471-2105
- GRAUL L ET AL: "Montelukast sodium, MK-476, MK-0476, L-706631, Singulair", DRUGS OF THE FUTURE, PROUS SCIENCE, ES, vol. 22, no. 10, 1 January 1997 (1997-01-01), page 1103, XP008082254, ISSN: 0377-8282
- IVANO EBERINI ET AL: "In silico identification of new ligands for GPR17: a promising therapeutic target for neurodegenerative diseases", JOURNAL OF COMPUTER-AIDED MOLECULAR DESIGN, KLUWER ACADEMIC PUBLISHERS, DO, vol. 25, no. 8, 9 July 2011 (2011-07-09), pages 743-752, XP019955577, ISSN: 1573-4951, DOI: 10.1007/S10822-011-9455-8

## Description

### Field of invention

The present invention concerns compounds able to modulate the activity of the GPR17 receptor in a highly specific way, and their use in the treatment and diagnosis of diseases and disorders involving the activation of said receptor, in particular their use for neuroprotective and/or reparatory purposes in cerebral, cardiac and renal ischaemia, in traumatic brain injury and in demyelinating diseases such as multiple sclerosis, schizophrenia, depression, Alzheimer's disease, Alzheimer-like dementia, Parkinson's disease, Huntington's chorea, amyotrophic lateral sclerosis (ALS) and all other neuroinflammatory disorders.

### State of the art

The possibility of modulating the brain tissue remodelling/repair processes through specific targets opens up some interesting therapeutic prospects for treating brain deficits and diseases that have so far been considered incurable. The medical profession currently has resources available which limit the progress of neurodegeneration following trauma, acute or chronic diseases, but is unable to induce regression of brain degeneration processes and reconstruction of the brain circuits. Diseases like cerebral ischaemia and multiple sclerosis are consequently still among the main causes of death and permanent disability in the Western world, including Europe, Japan, the USA and Canada.

Recently, GPR17, a novel P2Y-like receptor belonging to the superfamily of G-protein coupled receptors (GPCRs), was identified as a new promising therapeutic target for the manipulation of brain dysfunctions resulting from ischaemia, trauma, acute or chronic diseases characterised by dysfunction of myelin, the sheath surrounding the nerve extensions that allows the transmission of impulses. GPR17 is a dual receptor responding to two distinct classes of endogenous ligands: extracellular nucleotides and cysteinyl leukotrienes (CysLTs). During an ischaemic event, a massive accumulation of extracellular nucleotides and CysLT takes place at the time of the damage, suggesting that coordinated action by both classes of molecule induces the inflammatory response and the consequent repair/remodelling processes [Ciana et al., 2006, EMBO J 25:4615]. GPR17, the common molecular target of these two classes of molecule, is one of the main players governing these mechanisms, which participate in both the development of the damage and its subsequent repair. In fact, data from some of the inventors of the present application, reported in WO2006/045476 A2, demonstrates that *in vivo* specific inhibition of GPR17 by antagonist ligands or antisense oligonucleotides for the receptor expression reduces the progress of ischaemic damage in a rat focal ischaemia model. Together with the damage worsening effect attributed to GPR17, which is observed in the first acute stages of the ischaemic response (according to recent data, this is due to dysregulation of the receptor under these pathological conditions), it has been observed that GPR17 can produce beneficial effects in the later stages by stimulating the endogenous repair mechanisms typical of some specific cell populations present in the ischaemic regions [Lecca et al. 2008, PLoSONE 3:e3579]. In particular, it has been postulated that GPR17 plays a part in controlling the transition of oligodendrocyte precursor cells into mature oligodendrocytes with a myelinating phenotype [Chen et al., 2009, Nat Neurosci 12:1398] which have the potential to repair damage, thus promoting reconstruction of the damaged myelin sheath. Detailed studies of GPR17 expression in oligodendrocyte precursor cells maintained *in vitro* at various stages of differentiation have demonstrated that the receptor appears very soon in those cells, and reaches its maximum degree of expression when the precursor ceases to proliferate, leaves the cell cycle and commences its irreversible differentiation. However, at later stages (immature pre-oligodendrocyte), GPR17 must be deactivated so that the cells can complete their terminal differentiation [Fumagalli et al., 2011, J Biol Chem 286:10593]. GPR17 therefore identifies a very precise stage in oligodendrocyte differentiation. The data obtained *in vitro* also demonstrates that GPR17 agonists (e.g. UDP-glucose) promote cell maturation, while antagonists (e.g. cangrelor) delay it. Moreover, early knock-down of GPR17 at the first differentiation stages using small silencer RNAs completely inhibits cell maturation. GPR17 is therefore present at a very early stage on these precursors, and is needed to direct their differentiation. These conclusions are confirmed by *in vivo* studies conducted on developing and adult rodents [Boda et al., 2011, Glia doi: 10.1002/glia.21237]. Finally, confirming the crucial role of GPR17 in the myelinating process, in the *in vivo* model of focal demyelination induced by ethidium bromide, an accumulation of activated oligodendroglial precursors expressing the receptor was observed around the demyelinating lesions [Boda et al., Glia doi: 10,1002/glia.21237].

GPR17 therefore represents a damage sensor which is activated as a result of trauma/damage, and a promoter of the remodelling/repair process. The same mechanisms have been observed not only in the brain, but also in the bone marrow in a typical spinal lesion model, and in the focal demyelination model mentioned above. GPR17 is therefore a promising target for identifying new treatment strategies to repair the lesions present in ischaemia and demyelinating diseases. Highly selective GPR17 ligands are needed for this purpose, as those already known compete for other receptors correlated with it.

No selective ligands for GPR17 are known at present. The molecules currently available that are able to modulate its activity are shared with other correlated receptors, namely the P2Y and CysLT receptors, and belong clinically to classes of agonist/antagonist ligands which have been used in treatment for years. The absence of new, specific pharmacological tools represents an obstacle to exploiting the therapeutic potential of GPR17 without interfering with other physiological mechanisms.

There is currently no therapeutic treatment for the ischaemic disorders in which GPR17 is involved (which are due to both neuron and myelin damage) that acts on the mechanism underlying the damage.

Cerebral ischaemia is the third-highest cause of death in the European Union, with 650,000 cases a year, and the main cause of long-term disability.

Multiple sclerosis, a chronic progressive disease caused by destruction of the myelin sheath, is also one of the most widespread diseases of the central nervous system, affecting approximately 1.3 million people worldwide, 400,000 them in Europe alone. This incidence is aggravated by the fact that unlike ischaemic disorders, multiple sclerosis affects very young people who need chronic treatment, with a major social impact and financial burden on the National Health Service, The first-choice pharmacological treatments for this disease are aspecific, and not sufficient to control its development. For example, although the medicaments currently available, which belonging to the therapeutic category of immunomodulators, present modest efficacy in acute treatments, they are unable to cure the disease, only to slow its progress. Moreover, all the available medicaments cause significant, invalidating local and systemic adverse effects.

In view of the established role of GPR17 in the formation of the myelin sheath [Lecca et al. 2008, PLoSONE 3:e3579, Chen et al., 2009, Nat Neurosci 12:1398], its new specific ligands represent powerful pharmacological agents for the treatment of cerebral ischaemia and multiple sclerosis and treatment of other types of disorder characterised by demyelination for which no cure is yet available, such as schizophrenia, depression, Alzheimer's disease, Alzheimer-like dementia, Parkinson's disease, amyotrophic lateral sclerosis (ALS) and Huntington's chorea.

The use of highly specific modulators could make a considerable impact on the treatment of these human disorders, without the risk of interfering with the physiopathological functions mediated by other similar receptors.

The only molecules currently on the market that are able to inhibit GPR17 activity belong to the lukast family (montelukast, pranlukast and zafirlukast) and were marketed as CysLT receptor antagonists, ie. only with an anti-asthma indication. Another known GPR17 antagonist is cangrelor, an adenosine triphosphate derivative which has not completed phase 3 clinical trials and was developed as an antagonist of some P2Y receptors for use as a platelet aggregation inhibitor. Ticagrelor, an alternative to cangrelor, was recently approved by the European Medicines Agency (EMA) (2 December 2010) and the US Food and Drug Administration (20 July, 2011) as a novel platelet aggregation inhibitor.

### Description of the invention

The present invention relates to compounds, which are highly specific for GPR17, identified according to their ability to interact with the molecular structure of the binding site present on the GPR17 receptor. The modelling of said site was performed by means of an *in silico* experimental procedure based on a comparative chimeric modelling method developed by the inventors of the present application [Eberini et al., 2011, J Comput Aided Mol Des 25:743]. This allowed the identification of compounds which interact with the GPR17 receptor in an optimised way, in both geometrical and energy terms. The pharmacologically active compounds were identified on this basis.

The subject of the invention is a compound selected from the group consisting of:
2-[[5-(2-methoxyphenyl)-4-(4-methoxyphenyl)-4H-1,2,4-triazol-3-yl]thio]-N-phenylpropanamide of formula (I): 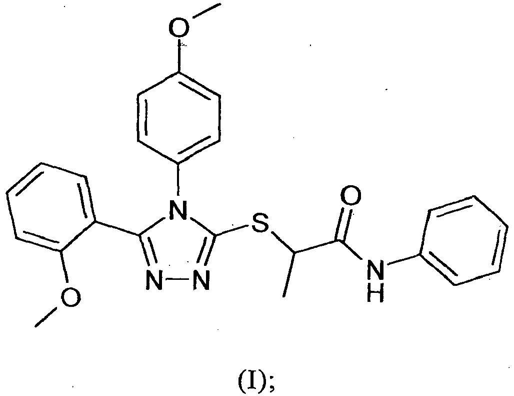
6-methoxy-3-[[1-(2-methoxyethyl)-1H-tetrazol-5-yl][(2-phenylethyl)(phenylmethyl)-amino]methyl]-2(1H)-quinolinone of formula (II): 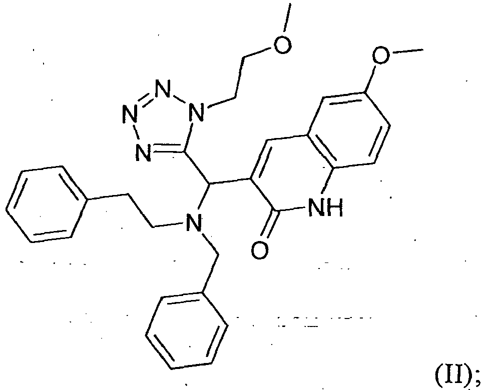
4-amino-N5-(2-furanylmethyl)-N5-[1-(4-hydroxyphenyl)-2-[[(4-methoxyphenyl)-methyl]amino-2-oxoethyl]-3,5-isothiazole dicarboxamide of formula (III): 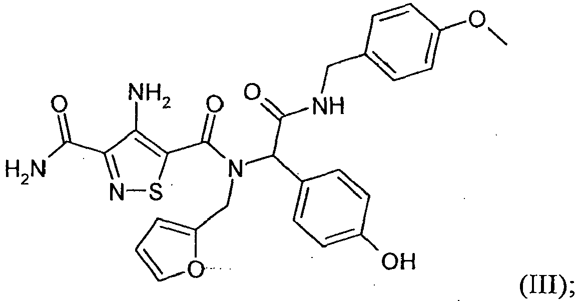
N-[1-(4-fluorophenyl)-2-oxo-2-[[(tetrahydro-2-furanyl)methyl]amino]ethyl]-N-(4-methoxyphenyl)-1H-benzotriazol-1-acetamide of formula (IV): 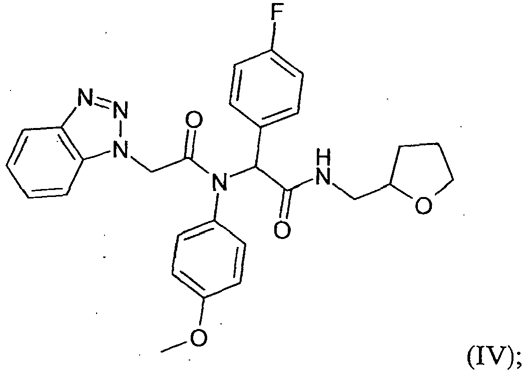
4-[3-[(2-chlorophenyl)methyl]-6,7-dihydro-7-oxo-3H-1,2,3-triazole[4,5-d]pyrimidin-5-yl]-N-[4-(trifluoromethyl)phenyl]-1-piperidinecarboxamide of formula (V): 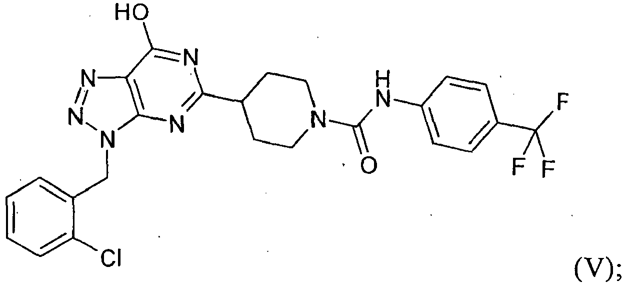
or a salt, isomer, single enantiomer, racemate or tautomer thereof, as therapeutic agent.

The compounds according to the invention are known and commercially available, but no pharmacological activity or therapeutic application is reported for them.

The compound of formula (I) has *CAS Registry Number* 483283-39-2 (sold by Asinex, code ASN02563583).

The compound of formula (II) has *CAS Registry Number* 570365-12-7 (sold by Asinex, code ASN04421891).

The compound of formula (III) has *CAS Registry Number* 1032654-11-7 (sold by Asinex, code ASN04450772).

The compound of formula (IV) has *CAS Registry Number* 1032892-26-4 (sold by Asinex, code ASN04885796).

The compound of formula (V) has *CAS Registry Number* 837404-68-9 (sold by Asinex, code ASN06917370).

The compounds of the invention can be prepared according to the synthesis procedures detailed in examples 1-6 of the present application.

Said compounds possess high power on GPR17, significantly greater than that of the endogenous ligands, and are not structurally correlated with the known ligands able to interact with GPR17, which are mainly agonists and antagonists of the P2Y and CysLT receptors related to GPR17.

The compounds according to the invention, when tested *in vitro* with the classic pharmacological assays, developed to determine the affinity/activity of molecules active on GPCRs, proved to be potent and effective modulators of GPR17 activity, with a high affinity for GPR17 (nanomolar, subnanomolar/picomolar IC50/EC50), which allows the specificity of action to be increased and any side effects due to the interaction with other P2Y or CysLT receptors to be minimised. The same compounds, tested in a pro-myelinating activity test [Fumagalli et al., 2011, J Biol Chem 286:10593] based on the use of oligodendrocyte precursor cell (OPC) cultures purified from postnatal rat brain maintained *in vitro,* promoted the maturation of the precursor cells to mature oligodendrocytes expressing basic myelin protein.

A further subject of the invention is therefore represented by a compound of formula (I), (II), (III), (IV) or (V), as defined above, as therapeutic agent for use in the treatment of disorders involving GPR17 receptor activation, in particular for the treatment of acute and chronic inflammatory, immune-system, cardiovascular, renal and neoplastic neurodegenerative diseases and dysfunctions.

According to a preferred aspect of the invention the compounds of formula (I), (II), (III), (IV) or (V), as defined above, are used as neuroprotective and/or reparatory agents in ischaemia, cerebral trauma and multiple sclerosis.

According to another preferred aspect of the invention, the compounds of formula (I), (II), (III), (IV) or (V) as defined above are used to treat a demyelinating disease such as multiple sclerosis, schizophrenia, depression, Alzheimer's disease, Alzheimer-like dementia, Parkinson's disease, Huntington's chorea, amyotrophic lateral sclerosis (ALS) or a neuro-inflammatory disease.

According to another preferred aspect, the compounds of formula (I), (II), (III), (IV) or (V) as defined above are used to treat cerebral, cardiac or renal ischaemia.

According to a particularly preferred aspect, the compounds of formula (I), (II), (III), (IV) or (V) as defined above are used to treat ischaemic brain damage.

The compounds according to the present invention can be administered in doses ranging from 0.01 mg to 10 mg per kg of body weight a day. A preferred method of administration uses a dose of approx. 0.5 mg to approx. 5 mg per kg of body weight a day, using unit doses which administer approx. 2 mg to approx. 200 mg of active substance in 24 hours to a patient weighing approx. 70 Kg. Such a method of administration can be adjusted to obtain a better therapeutic effect. For example, the doses can be adjusted on the basis of the patient's therapeutic situation.

The compounds according to the invention can be administered orally, intravenously, intramuscularly or subcutaneously.

For therapeutic use, the compounds according to the invention can be suitably formulated with physiologically acceptable excipients or carriers. The suitable pharmaceutical forms can vary according to the specific compound and the administration route. The dose of active ingredient will be determined on each occasion, on the basis of the severity of the disease to be treated and the patient's general condition. Suitable pharmaceutical compositions can be prepared according to the indications reported in Remington's Pharmaceutical Sciences, 18th ed., Mack Publishing Co.

The pharmaceutical compositions according to the present invention contain therapeutically effective quantities of at least one compound according to the invention, mixed with excipients compatible with pharmaceutical use.

The oral compositions generally contain an inert diluent or an edible carrier, and can be enclosed in gelatin capsules or compressed into tablets. Other possible forms for oral administration are capsules, pills, elixirs, suspensions and syrups.

Said tablets, pills, capsules and similar compositions can contain the following ingredients (in addition to the active compound): a binder such as microcrystalline cellulose, gum tragacanth or gelatin; a carrier such as starch or lactose, a disintegrating agent such as alginic acid, primogel, corn starch or the like; a lubricant such as magnesium stearate; a fluidifier such as colloidal silicon dioxide; a sweetener such as saccharose or saccharine or a flavouring such as mint flavouring, methyl salicylate or orange flavouring. When the selected composition is in capsule form, it can also contain a liquid carrier such as a fatty oil. Other compositions can contain various materials, e.g. coating agents (for tablets and pills) such as sugar or shellac. The material used to prepare the compositions should be pharmaceutically pure, and non-toxic at the doses used.

To prepare pharmaceutical compositions for parenteral administration, the active ingredient can be included in solutions or suspensions, which can also contain the following constituents: a sterile diluent such as water for injectables, saline solution, oil, polyethylene glycol, glycerin, propylene glycol or other synthetic solvents; antibacterial agents such as benzyl alcohol; antioxidants such as ascorbic acid or sodium bisulphite; chelating agents such as ethylenediaminetetraacetic acid; buffers such as acetates, citrates or phosphates and agents to adjust the tonicity of the solution, for example sodium chloride or dextrose. The parenteral preparations can be enclosed in vials, disposable syringes, and glass or plastic bottles.

A further subject of the invention is therefore a pharmaceutical composition containing a compound of formula (I), (II), (III), (IV) or (V) as defined above, in admixture with a suitable pharmaceutically acceptable carrier or excipient.

Further subjects of the invention relate to a compound of formula (I), (II), (III), (IV) or (V) as defined above for diagnostic use and for studying the function of the GPR17 receptor under physiological or pathological conditions.

The invention will now be illustrated by the following examples and the annexed figures.

### Example 1 - Synthesis of 2-[[5-(2-methoxyphenyl)-4-(4-methoxyphenyl)-4H-1,2,4-triazol-3-yl]thio]-N-phenylpropanamide of formula (I)

### a) Preparation of 4-(4-methoxyphenyl)-5-(2-methoxyphenyl)-4H-[1,2,4]triazol-3-thiol.

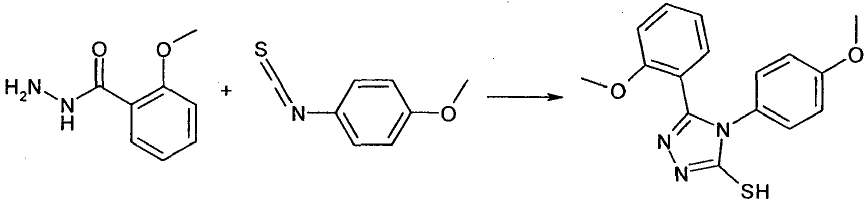

2-methoxybenzoyl hydrazide (Lancaster; 0.2 mol) was dissolved in ethanol (100 mL). 4-methoxyphenyl isothiocyanate (Acros; 0.22 mol) was added to this solution. The mixture was maintained under reflux stirring for 8 hours. A control TLC performed after this time showed the formation of the thioureido intermediate. A 15% aqueous solution of KOH (0.24 mol.) was added at this point. The resulting mixture was reflux heated under stirring for 20 h. On completion of the reaction (TLC control) the reaction mixture was concentrated at low pressure. The residue obtained was diluted with water (200 mL) and acidified at pH 4.5 with 2N HCl. The precipitate formed was separated by filtration, washed with water and dried. The crude product was crystallised with dimethylformamide, giving 4-(4-methoxyphenyl)-5-(2-methoxyphenyl)-4H-[1,2,4]triazol-3-thiol (46 g, 74%).

### b) Preparation of 2-chloro-N-phenylpropionamide.

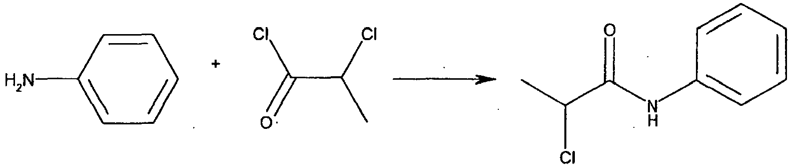

2-chloropropionyl chloride (1.1 mol) was added to a solution of phenylamine (1 mol) in approx. 200-300 mL of anhydrous acetonitrile (or anhydrous DMF). The addition was performed in small portions to prevent boiling and sputtering of the reaction mixture. The latter is then reflux heated (or, when the reaction solvent is DMF, stirred at 100°C) until the end of development of gaseous HCl and the disappearance of the amine spot in TLC. The reaction mixture was then allowed to return to ambient temperature, poured into cold water (1 L) and left under stirring for as long as required for the product to solidify. The precipitate formed was separated by filtration, washed with water and dried. When, after pouring and stirring in cold water, the product did not solidify but remained in oil form, the oily phase of the amide was separated, the aqueous phase was extracted with ethyl ether and the combined organic phases were dried on Na₂SO₄ and concentrated at low pressure, and the residue was crushed on ethyl ether to give 2-chloro-N-phenylpropionamide (130 g, 71%) in solid form.

### c) 2-[[5-(2-methoxyphenyl)-4-(4-methoxyphenyl)-4H-1,2,4-triazol-3-yl]thio]-N-phenylpropanamide (I)

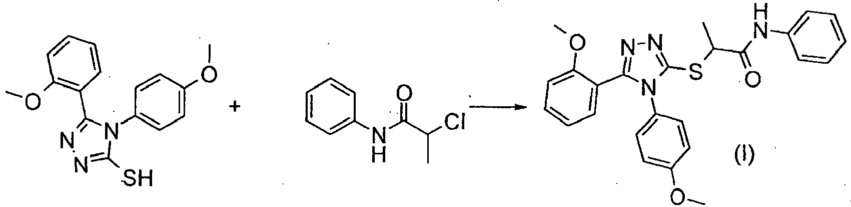

The 4-(4-methoxyphenyl)-5-(2-methoxyphenyl)-4H-[1,2,4]triazol-3-thiol obtained in step a) (1 mmol) was suspended in ethanol (8 ml). A 15% aqueous solution of KOH (0.9 mmol) and triethylamine (0.3 mmol) were added to the solution obtained. The mixture was heated to 50°C and the 2-chloro-N-phenylpropionamide obtained in step b) (1.1 mmol) was added. The mixture obtained was reflux heated for 4 h. After cooling to room temperature, the mixture was diluted with water (approx. 5-10 mL). The precipitate formed was separated by filtration, washed with an ethanol/water mixture (1/1) and dried to give a crude product. Recrystallisation with ethanol/water (1/1) (or with acetone/water 1/1) supplied pure 2-[[5-(2-methoxyphenyl)-4-(4-methoxyphenyl)-4H-1,2,4-triazol-3-yl]thio]-N-phenylpropanamide (I) (315 mg, 66%).

### Example 2 - Synthesis of 6-methoxy-3-[[1-(2-methoxyethyl)-1H-tetrazol-5-yl][(2-phenylethyl)(phenylmethyl)amino]methyl]-2(1H)-quinolinone of formula (II)

### a) Preparation of 6-methoxy-2-oxo-1,2-dihydroquinolin-3-carboxaldehyde

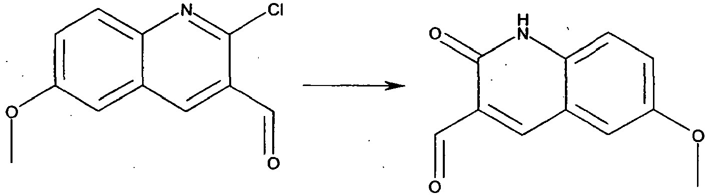

A mixture of 2-chloro-6-methoxyquinolin-3-carboxaldehyde (0.1 mol) and water (0.5 mol) in acetic acid (200 mL) was reflux heated stirring for 8 h. The reaction was monitored by TLC. The reaction mixture was cooled. The precipitate formed was collected by filtration, washed with acetone and air-dried to give 6-methoxy-2-oxo-1,2-dihydroquinolin-3-carboxaldehyde (17 g, 83%).

### b) 6-methoxy-3-[[1-(2-methoxyethyl)-1H-tetrazol-5-yl][(2-phenylethyl)-(phenylmethyl)amino]methyl]- 2(1H)-quinolinone (II)

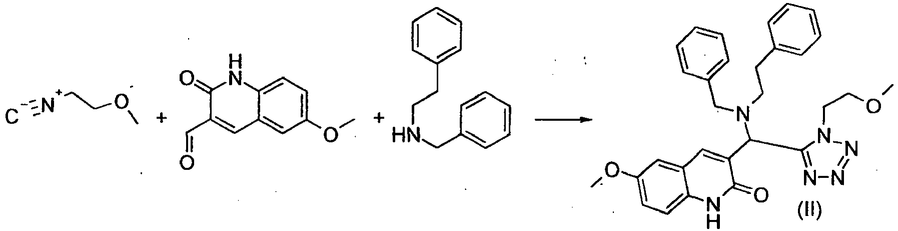

A mixture of the 6-methoxy-2-oxo-1,2-dihydroquinolin-3-carboxaldehyde obtained in step a) (2 mmol) and N-benzylphenylamine (Acros; 2 mmol) in methanol (10 mL) was heated at 50°C under stirring for 2-3 h. 2-methoxyethyl isonitrile (Zerenex; 2 mmol) and trimethylsilyl azide (3 mmol) were then added, and the mixture was heated under stirring at 50°C for 10 h. After the completion of the reaction (TLC valuation), the solvent was removed by low-pressure distillation. The residue obtained was purified by column chromatography (silica gel, eluent dichloromethane) to give pure 6-methoxy-3-[[1-(2-methoxyethyl)-1H-tetrazol-5-yl[(2-phenylethyl)(phenylmethyl)amino]methyl]- 2(1H)-quinolinone (II) (331 mg, 35%).

### Example 3 - Synthesis of 4-amino-N5-(2-furanylmethyl)-N5-[1-(4-hydroxyphenyl)-2-[[(4-methoxyphenyl)methyl]amino-2-oxoethyl]-3,5-isothiazole-dicarboxamide of formula (III)

### a) Preparation of 2-cyano-2-[(Z)-hydroxyimino]acetamide

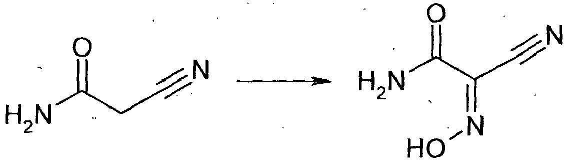

A saturated solution of sodium nitrite (172.5 g, 2.5 mol) in water was added drop by drop at 5-10°C to a solution of 2-cyanoacetamide (84 g, 1 mol) in acetic acid (250 mL). The reaction mixture was maintained under stirring at 10°C for 12 h. The mixture obtained was filtered, and the solid collected was dried to give 2-cyano-2-[(Z)-hydroxyimino]acetamide (88 g, 78%).

### b) Preparation of 2-cyano-2-[(Z)-tosyloxyimine]acetamide

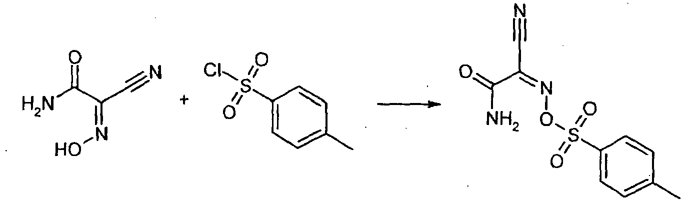

4-methylbenzenesulphonyl chloride (51.3 g, 0.27 mol) was added in portions, at 5°C, to a solution of 2-cyano-2-[(Z)-hydroxyimino]-acetamide (28.25 g, 0.25 mol) in anhydrous pyridine (100 mL). The reaction mixture was maintained under stirring at room temperature for 6 h, left to stand overnight and then treated with ethanol (300 mL). The mixture obtained was filtered. The solid recovered was washed with cold ethanol and dried, to give pure 2-cyano-2-[(Z)-tosyloxyimino]acetamide (54 g, 81%).

### c) Preparation of ethyl 4-amino-3-carbamoylisothiazol-5-carboxylate

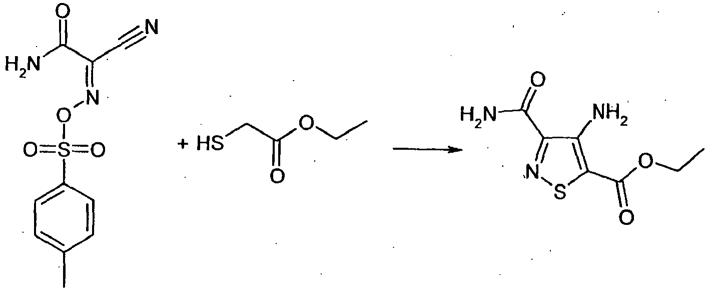

Piperidine (8.5 g, 0.1 mol) was dripped at 5°C into a solution of 2-cyano-2-[(Z)-tosyloxyimino]acetamide (53.4 g, 0.2 mol) and ethyl 2-mercaptoacetate (25 g, 0.21 mol) in ethanol (250 mL). The reaction mixture was maintained under stirring at room temperature for 6 h, and then left to stand overnight. The reaction mixture was cooled to -10°C. The precipitate formed was recovered by filtration, washed with cold ethanol (100 mL) and air-dried to give ethyl 4-amino-3-carbamoylisothiazol-5-carboxylate (25 g, 58%).

### d) Preparation of 4-amino-3-carbamoylisothiazol-5-carboxylic acid

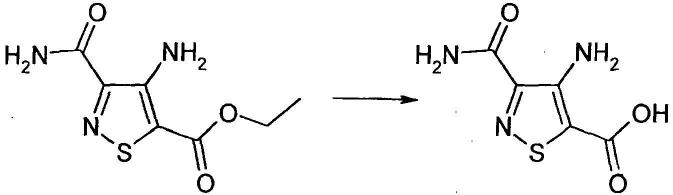

A solution of NaOH (8 g, 0.2 mol) in water (60 mL) was added drop by drop to a solution of ethyl 4-amino-3-carbamoylisothiazol-5-carboxylate (21.5 g, 0.1 mol) in ethanol (100 mL). The reaction mixture was left under stirring overnight. It was then evaporated, the residue obtained was dissolved in water, and the aqueous phase was extracted with ethyl acetate. The aqueous phase was then acidified to pH 4 with concentrated HCl. The product precipitated as a white powder that was recovered by filtration and dried, to give pure 4-amino-3-carbamoylisothiazol-5-carboxylic acid (15.5 g, 83%).

### e) 4-amino-N5-(2-furanylmethyl)-N5-[1-(4-hydroxyphenyl)-2-[[(4-methoxyphenyl)methyl]amino]-2-oxoethyl]-3,5-isothiazole dicarboxamide (III)

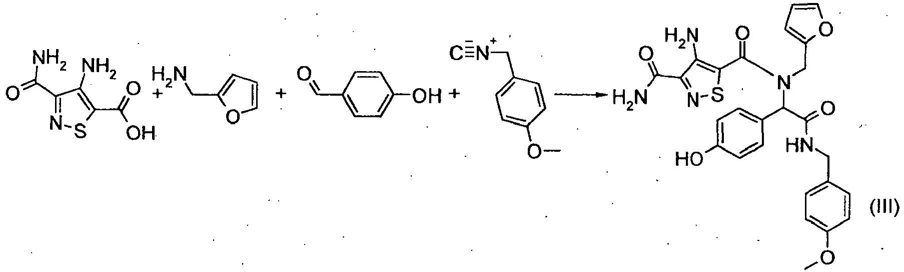

A mixture of 4-hydroxybenzaldehyde (Acros; 2 mmol) and furfurylamine (Acros; 2 mmol) in methanol (10 mL) was maintained under stirring at 50°C for 2-3 h. The 4-methoxybenzyl isonitrile (Fluorochem; 2 mmol) and 4-amino-3-carbamoylisothiazol-5-carboxylic acid prepared in step c) (2 mmol) were then added, and the mixture was left under stirring at ambient temperature for 24 h. After completion of the reaction (evaluated by TLC), the solvent was removed at low pressure and the residue obtained was purified by column chromatography (silica gel, eluent dichloromethane), to give pure 4-amino-N5-(2-furanylmethyl)-N5-[1-(4-hydroxyphenyl)-2-[[(4-methoxyphenyl)methyl]amino-2-oxoethyl]- 3,5-isothiazole dicarboxamide (III) (522 mg, 54%).

### Example 4 - Synthesis of N-[1-(4-fluorophenyl)-2-oxo-2-[[(tetrahydro-2-furanyl)methyl]amino]ethyl]-N-(4-methoxyphenyl)-1H-benzotriazol-1-acetamide of formula (IV)

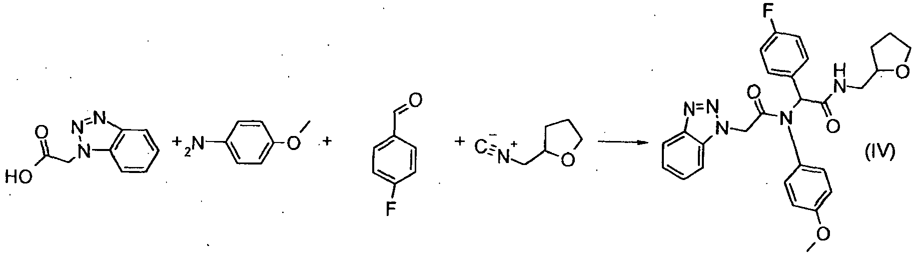

A mixture of 4-fluorobenzaldehyde (TCI-Europe; 2 mmol) and p-anisidine (Acros; 2 mmol) in methanol (10 mL) was maintained under stirring at 50°C for 2-3 h. 2-tetrahydrofurfuryl isonitrile (Zerenex; 2 mmol) and (1H-benzotriazol-1-yl)acetic acid (Matrix; 2 mmol) were then added, and the mixture obtained was maintained under stirring at room temperature for 24 h. After completion of the reaction (TLC evaluation), the solvent was removed at low pressure. The residue was purified by column chromatography (silica gel, eluent dichloromethane), to give pure N-[1-(4-fluorophenyl)-2-oxo-2-[[(tetrahydro-2-furanyl)methyl]amino]ethyl]-N-(4-methoxyphenyl)-1H-benzotriazol-1-acetamide (IV) (505 mg, 51%).

### Example 5 - Synthesis of 4-[3-[(2-chlorophenyl)methyl]-6,7-dihydro-7-oxo-3H-1,2,3-triazole[4,5-d]pyrimidin-5-yl]-N-[4-(trifluoromethyl)phenyl]-1-piperidinecarboxamide of formula (V)

### a) Preparation of 1-azidomethyl-2-chlorobenzene

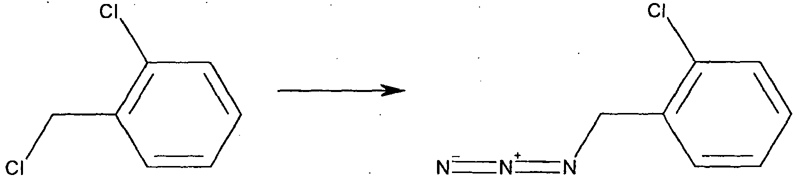

Sodium azide (200 mmol) was added to a solution of 2-chlorobenzyl chloride (200 mmol) in anhydrous acetonitrile (200 mL). The mixture was left under stirring at 60°C for 2 h (monitored with TLC) and cooled to room temperature. The solvent was removed at low pressure. The residue obtained was diluted with water (300 mL) and extracted with dichloromethane (2x150 mL). The combined organic phases were dried with Na₂SO₄, concentrated at low pressure and dried to give 1-azidomethyl-2-chlorobenzene (26 g, 77%).

### b) Preparation of tert-butyl 4-[3-(2-chlorobenzyl)-7-hydroxy-3H-[1,2,3]triazole[4,5-d]pyrimidin-5-yl]-1-piperidinecarboxylate

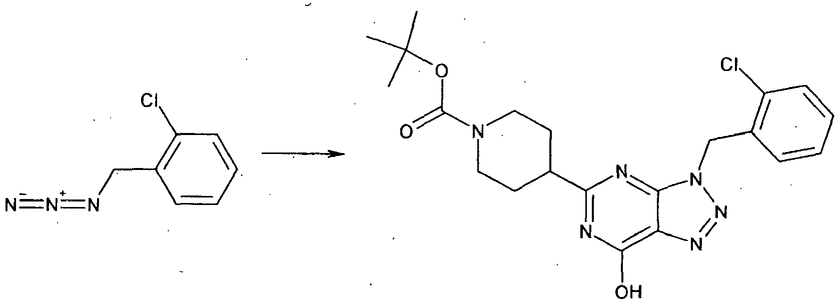

2-cyanoacetamide (100 mmol) was added to a solution of sodium (400 mmol) in anhydrous isopropanol (1 L) maintained under stirring. The mixture was reflux heated, and 1-azidomethyl-2-chlorobenzene (100 mmol) was added. The mixture obtained was maintained under stirring reflux for 2 h, and piperidin-1,4-dicarboxylic acid 1-tert-butyl ester 4-ethyl ester (100 mmol) was then added; reflux heating continued under stirring for another 2 h, the progress of the reaction being monitored with TLC. The solvent was removed at low pressure. The residue obtained was diluted with water (400 mL), leading to the separation of a solid. The solid was recovered by filtration and recrystallised from dioxane to give tert-butyl 4-[3-(2-chlorobenzyl)-7-hydroxy-3H-[1,2,3]triazole[4,5-d]pyrimidin-5-yl-1-piperidinecarboxylate (27 g, 61%).

### c) Preparation of 3-(2-chlorobenzyl)-5-(piperidin-4-yl)-3H-[1,2,3]triazole[4,5-d]pyrimidin-7-ol hydrochloride

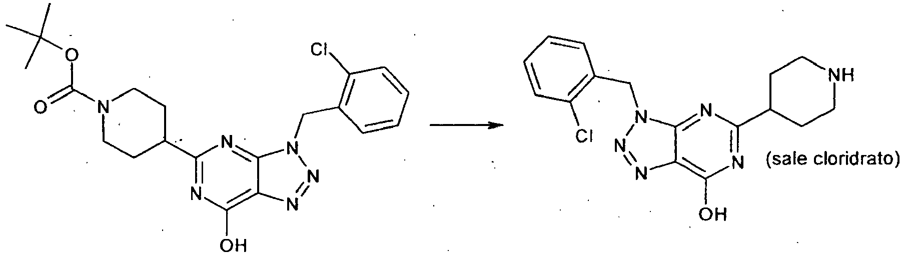

The tert-butyl 4-[3-(2-chlorobenzyl)-7-hydroxy-3H-[1,2,3]triazole[4,5-d]pyrimidin-5-yl]-1-piperidinecarboxylate prepared in step b) (50 mmol) was dissolved in a 14% solution of gaseous HCl in dioxane (100 mL). The solution was maintained under reflux stirring for 5 h and then cooled to room temperature to give a solid. The solid was collected by filtration, washed with hot acetone and dried to give 3-(2-chlorobenzyl)-5-(piperidin-4-yl)-3H-[1,2,3]triazole[4,5-d]pyrimidin-7-ol in the form of hydrochloride (16 g, 85%). The product obtained was converted to the free base by treatment with a 30% aqueous solution of potassium hydroxide, followed by extraction with dichloromethane and evaporation of the solvent. The resulting free base was used in the next synthesis step.

### d) 4-[3-[(2-chlorophenyl)methyl]-6,7-dihydro-7-oxo-3H-1,2,3-triazole[4,5-d]pyrimidin-5-yl]-N-[4-(trifluoromethyl)phenyl]-1-piperidinecarboxamide (V)

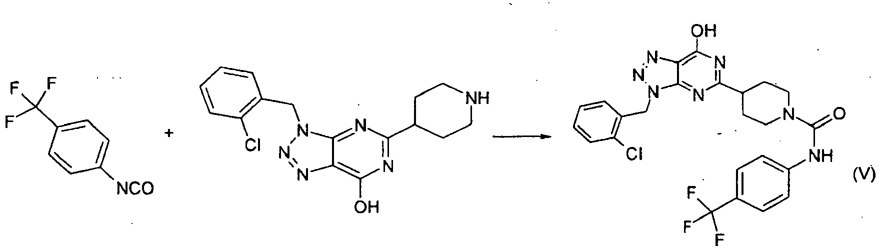

4-trifluorophenyl isocyanate (1.1 mmol) was added to a solution of 3-(2-chlorobenzyl)-5-(piperidin-4-yl)-3H-[1,2,3]triazole[4,5-d]pyrimidin-7-ol) (1 mmol) in acetonitrile (5 mL). The reaction mixture was reflux heated for 30 min. and then cooled to room temperature, treated with a saturated aqueous solution of NaHCO₃ (15 mL) and extracted with dichloromethane (2x10 mL). The combined organic phases were dried on Na₂SO₄ and concentrated at low pressure until dry to give a crude product. Said product was purified by column chromatography (silica gel; eluent hexane/ethyl acetate) to give pure 4-[3-[(2-chlorophenyl)methyl]-6,7-dihydro-7-oxo-3H-1,2,3-triazole[4,5-d]pyrimidin-5-yl]-N-[4-(trifluoromethyl)phenyl]-1-piperidinecarboxamide (V) (245 mg, 58%).

### Example 6 - In vitro evaluation of the activity of the compounds according to the invention by functional pharmacological tests for the determination of affinity parameters such as EC50/IC50 and efficacy (A: [³⁵S]GTPγS binding; B: pro-myelinating properties on oligodendrocyte precursor cell (OPC) cultures

A- The compounds according to the invention were evaluated for their functional activity on GPR17 using a well-known assay for GPCRs, the [³⁵S]GTPγS binding assay, which is based on the ability of the agonist to increase radioactive GTP binding when a GPCR is activated. The antagonist activity is evaluated according to the ability to counteract the increased [³⁵S]GTPγS binding induced by the LTC₄ reference agonist.

### Preparation and transfection of cell cultures

Human 1321N1 cells were cultured as described [Fumagalli et al., 2004, Biochem Pharmacol 68:113]. For the [³⁵S]GTPγS assay, 10⁶ 1321N1 cells were seeded on 75cm² flasks and transfected by the calcium phosphate precipitation method, with the vector pcDNA3.1 containing the construct encoding for human receptor GPR17, and with the empty vector pcDNA3.1 as control [Fumagalli et al., 2004, Biochem Pharmacol 68:113].

### [³⁵S]GTPγS binding assay

The activation of GPCRs by agonists stimulates the dissociation of GDP from G-protein, thus increasing GTP binding in its place. This increase in GTP binding to G-protein can be quantified *in vitro* with [³⁵S]GTPγS. The effect of GPCR activation on [³⁵S]GTPγS binding is catalytic, because it increases the [³⁵S]GTPγS binding rate rather than [³⁵S]GTPγS binding equilibrium. Consequently, the [³⁵S]GTPγS binding assay is intrinsically a functional concentration-response curve, not an equilibrium binding assay [Lazareno, 1999, Methods Mol Biol 1106:231; Milligan, 2003, Trends Pharmacol Ski 24:87]. For some GPCRs, the binding stimulated by the agonist (ie. the difference between GTP binding in the presence and absence of the agonist) increases with time, peaks and then falls to zero; optimal increases in [³⁵S]GTPγS binding induced by the agonist are often obtained before equilibrium is attained, rather than at equilibrium. Preliminary studies of kinetic association constants performed on 1321N1 cells to define the optimal binding conditions have already been published [Ciana et al., 2006, EMBO J 25:4615]. The control and transfected cells were homogenised in 5 mM Tris-HCl and 2 mM EDTA (pH 7.4) and centrifuged at 48000 g for 15 min at 4°C. The resulting pellets (plasma membranes) were washed in 50 mM Tris-HCl and 10 mM MgCl₂ (pH 7.4) and stored at -80°C until use. The [³⁵S]GTPγS binding stimulated by the nucleotides in cell membranes expressing the human receptor was performed as previously described [Fumagalli et al., 2004, Biochem Pharmacol 68:113; Ciana et al., 2006, EMBO J 25:4615 and Calleri et al., 2010, J Med Chem 53:3489].

### B- Pro-myelinating activity assay

### Preparation of primary cultures of oligodendrocyte precursor cells (OPCs)

OPCs were isolated from mixed glial cultures of Sprague-Dawley rat cortex at the second postnatal day, using the method devised in our laboratory and previously described in detail [Fumagalli et al., 2011, J Biol Chem 286:10593]. The OPCs were cultured on plates containing 24 mm slides coated with poly-D,L-ornithine in Neurobasal medium with 2% of B27 supplement (Invitrogen), L-glutamine (2 mM) and enriched with growth factors PDGF and betaFGF (10 ng/ml) to promote proliferation. In order to promote differentiation, after 24 h, the culture medium was replaced with Neurobasal with 2% B27 without growth factors and with the addition of triiodothyronine T₃ (400 ng/ml).

### Pharmacological treatments

On day 5 the OPCs were placed in differentiating medium containing T₃ with the 5 test compounds at the final concentrations of 1 nM (compounds of formula (I) and (V)) or 10 nM (compounds of formula (II), (III) and (IV)) or, in parallel samples, with the known reference endogenous agonists LTD₄ (10 nM) and UDP-glucose (100 µM).

### Pro-myelinating activity assay

To check whether the new ligands of formula (I), (II), (III), (IV) and (V) can promote differentiation of OPCs, as already described for endogenous agonists [Fumagalli et al., 2011, J Biol Chem 286:10593], the degree of maturity of the cultures was analysed 48 h after treatment by measuring the expression of a specific marker for terminal oligodendroglial differentiation, the myelin basic protein (MBP). The ability of the agonists to promote myelination was evaluated by measuring the increase in the number of cells positive for the MBP marker present in the culture compared with the control treated with the carrier alone. In particular, 48 h after treatment with the agonists, the cells were fixed in 4% paraformaldehyde in 0.1 M PBS and stained by immunocytochemistry using an antibody specific for MBP. Staining was performed according to the following procedure: after 30 min blocking of the aspecific sites in GSDB buffer (450 mM NaCl, 20 mM of phosphate buffer 240 mM, pH 7.4, 15% goat serum, 0.3% Triton X-100), the slides were incubated for 2.5 h at room temperature with rat anti-MBP primary antibody (Chemicon, 1:200 in GSDB). The cells were then incubated for 1 h at room temperature with goat anti-mouse secondary antibody conjugated with Alexa Fluor 555 (1:600 in GSDB; Molecular Probes, Invitrogen). Finally, the cell nuclei were stained with the fluorescent nuclear marker Hoechst 33258 for 20 min at room (1:10.000, Molecular Probes, Invitrogen). The slides were mounted with Dako Fluorescent Mounting Medium (Dako, Milan) on cover slips and analysed with an inverted optical microscope (AXIOVERT 200 M, Zeiss, Milan). The images were processed and acquired with Axiovision 4.4 software, in the rhodamine fluorescence channel for MBP. The effects of the treatments on OPC myelination were quantified by manual count using ImageJ software (Wayne Rasband, Research Services Branch of the National Institute of Mental Health of Bethesda, USA).

### Statistical analysis

The Graph-Pad Prism program was used for the analysis and graphic presentation of the [³⁵S]GTPγS binding data. All the data are shown as the mean ± SEM of three different experiments. The data were tested for statistical significance with the paired Student's t test or analysis of variance (one-way ANOVA), as appropriate. When significant differences were observed, the Newman-Keuls multiple comparison test (one-way ANOVA) was performed. A value of P < 0.05 was considered significant.

### Results

The results of the [³⁵S]GTPγS binding assay are set out in the Table.

All the compounds according to the invention present very high power in a narrow interval of concentrations, ranging from sub-nanomolar to nanomolar/picomolar.

All the compounds tested, apart from the compound of formula (III) (ASN04450772), exhibited efficacy comparable to or greater than that of the reference compound LTC₄. Only the compound of formula (III) presented efficacy (89.6%) less than that of LTC₄, suggesting that this compound may act as a partial agonist.

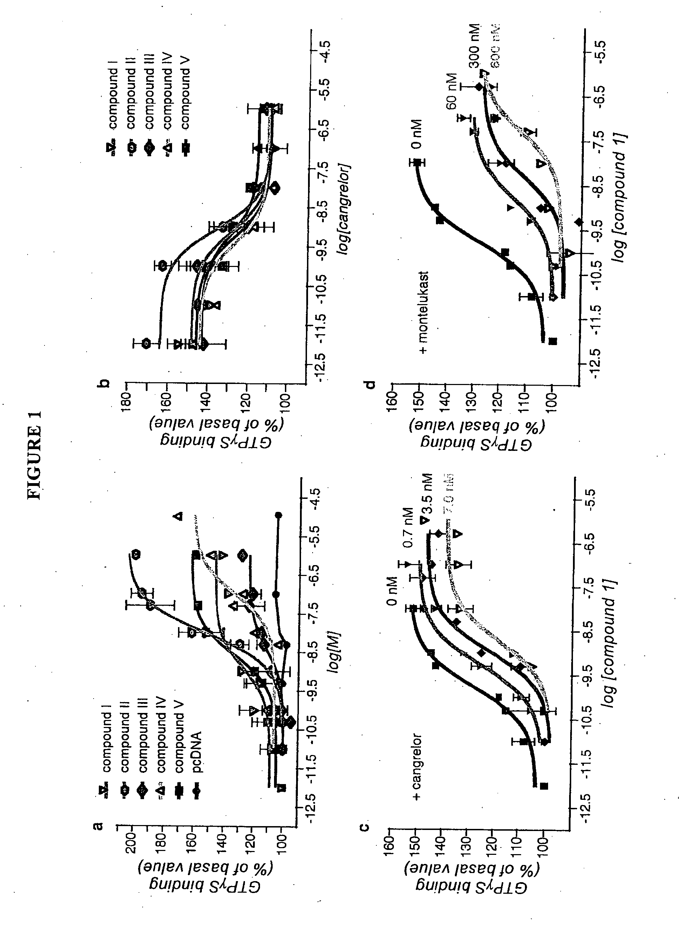

Figure 1, panel A shows the dose-response curves for the compounds according to the invention in the [³⁵S]GTPγS assay performed on 1321N1 cells that express GPR17. Reference compound LTC₄ was unable to induce a response in the cells transfected only with control vector pcDNA3.1 (black line), confirming that [³⁵S]GTPγS binding is mediated by the presence of GPR17. Figure 1, panel B shows the antagonist effect of cangrelor on the activation of GPR17 induced by the compounds according to the invention. The results demonstrate that all the compounds according to the invention are antagonised by cangrelor, an antagonist of P2Y_{12/13}, and GPR17, able to bind to the GPR17 uracil nucleotide binding site [Ciana et al., 2006, EMBO J 25:4615].

The concentration-response curves obtained for the most potent compound (compound of formula (I); ASN02563583) in the presence of different concentrations of cangrelor (Figure 1, panel C) are shifted to the right (increase in EC50), but no effect on efficacy was observed, suggesting that cangrelor is a competitive antagonist for the compound of formula I and that said two molecules compete for the same binding site on GPR17.

The same experiment was repeated with montelukast, a CysLTR1 antagonist [Brink, 2003, Adv Exp Med Biol 525:7] (Figure 1, panel D). The medicaments in the lukast class are known to act as inhibitors not only of CysLTR1, but also of GPR17 [Ciana et al., 2006, EMBO J 25:4615]. However, the dose-response curves for the compound of formula I, obtained in the presence of montelukast, show a reduction in the efficacy of the compound, suggesting non-competitive antagonism of montelukast on the effect of the compound of formula I. Said mechanism is probably exercised through an allosteric effect of montelukast on a binding site different from the one specific for uracil nucleotides to which the compound of formula I appears to bind.

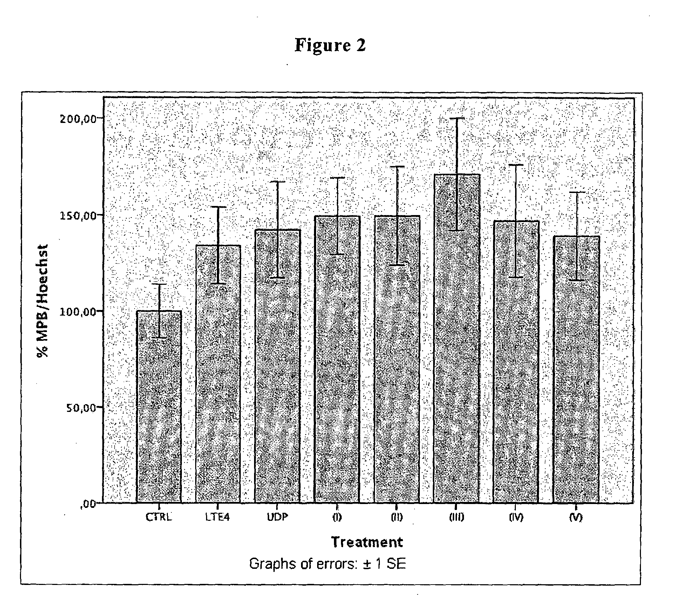

The results of the myelination test are shown in Figure 2 (pro-myelinating activities of the compounds according to the invention - assay conducted on OPC).

As expected, the endogenous agonists of GPR17 with known activity (LTE₄ and UDP) increase the number of mature cells in culture expressing MBP 48 h after the start of treatment.

All the compounds according to the invention also proved active, with activity ranging between 140 and 170%; although they were used at nanomolar concentrations (1 or 10 nM, as shown in Figure 2), they all presented activity comparable to or greater than that of reference standard compounds.

Compound (III), which proved the most active in this assay, acts as partial agonist on GPR17, and is therefore particularly interesting. It may represent the prototype of a new class of optimal protective agents able to develop their action in two successive stages: firstly by acting as antagonist in the initial phase of the damage and counteracting excessive activation of the GPR17 receptor in the presence of high local concentrations of its endogenous ligands, which are responsible for its dysregulation and cell death, and secondly by acting as agonist in the functional recovery phase, when receptor stimulation is necessary to activate the cell differentiation and remyelination process.

**Table - Pharmacological parameters for the compounds according to the invention determined in [³⁵S]GTPγS binding**

| Compound | **EC₅₀** | **Eₘₐₓ** | % **Emax** *vs* standard | **IC₅₀** Compound *vs* cangrelor |
|---|---|---|---|---|
| (V) **ASN06917370** | 268 ± 9 pM | 161.5 ± 2.4 | 111.5 ** | 0.78 ± 0.22 nM |
| (I) **ASN02563583** | 109 ± 28 pM | 145.6 ± 5.7 | 100.6 | 0.64 ± 0.19 nM |
| (III) **ASN04450772** | 1.18 ± 0.08 nM | 129.7 ± 0.7 | 89.6 *** | 0.51 ± 0.04 nM |
| (IV) **ASN04885796** | 2.27 ± 0.07 nM | 173.5 ± 0.5 | 119.8 *** | 0.48 ± 0.17 nM |
| (II) **ASN04421891** | 3.67 ± 0.51 nM | 206.8 ± 8.3 | 142.8 *** | 0.71 ± 0.09 nM |
| LTC₄ 100nM | | 144.8 ± 0.4 | 100 | |

| | | | | |
|---|---|---|---|---|
| ** P< 0.01 *vs* LTC₄ set to 100% *** P< 0.001 *vs* LTC₄ set to 100% | | | | |

## Claims

1. A compound selected from the group consisting of:
2-[[5-(2-methoxyphenyl)-4-(4-methoxyphenyl)-4H-1,2,4-triazol-3-yl]thio]-N-phenylpropanamide of formula (I): 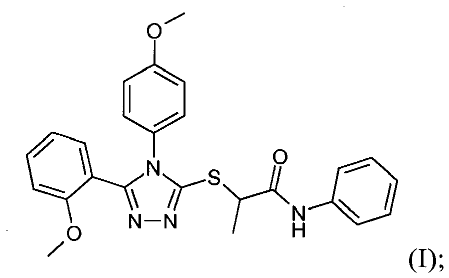
6-methoxy-3-[[1-(2-methoxyethyl)-1H-tetrazol-5-yl][(2-phenylethyl)-(phenylmethyl)amino]methyl]-2(1H)-quinolinone of formula (II): 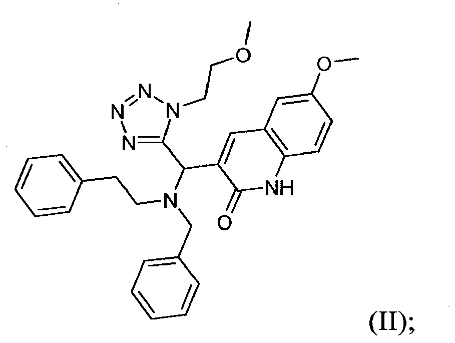
4-amino-N5-(2-furanylmethyl)-N5-[1-(4-hydroxyphenyl)-2-[[(4-methoxyphenyl)methyl]amino]-2-oxoethyl]-3,5-isothiazole dicarboxamide of formula (III): 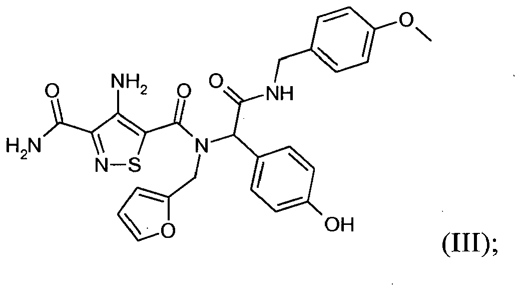
N-[1-(4-fluorophenyl)-2-oxo-2-[[(tetrahydro-2-furanyl)methyl]amino]ethyl]-N-(4-methoxyphenyl)-1H-benzotriazol-1-acetamide of formula (IV): 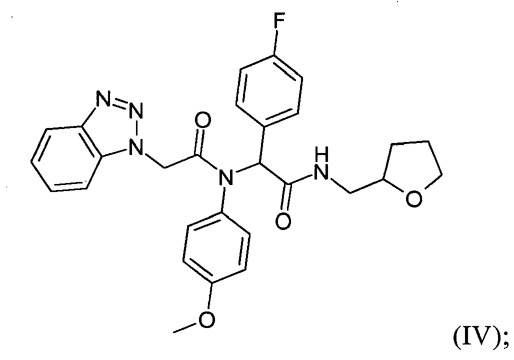
4-[3-[(2-chlorophenyl)methyl]-6,7-dihydro-7-oxo-3H-1,2,3-triazol[4,5-d]-pyrimidin-5-yl]-N-[4-(trifluoromethyl)phenyl]-1-piperidinecarboxamide of formula (V): 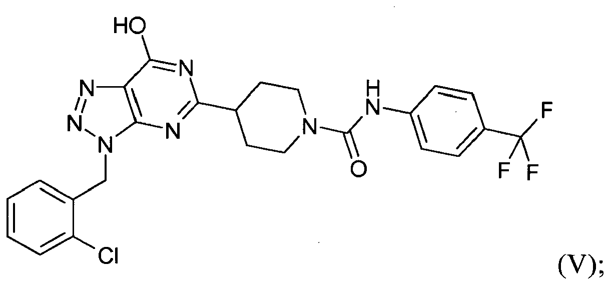
or a salt, isomer, single enantiomer, racemate or tautomer thereof, for use as a therapeutic agent.

2. A compound of formula (I), (II), (III), (IV) or (V), as defined in claim 1, for use in a method of treatment of diseases or dysfunctions involving GPR17 activation.

3. A compound according to claim 2, for use in a method of treatment of acute or chronic neurodegenerative inflammatory, immune-system, cardiovascular, renal and neoplastic diseases or dysfunctions.

4. A compound according to claims 2-3, for use as a neuroprotective agent or an agent for repairing ischaemia and cerebral trauma.

5. A compound according to claims 2-3 for use in a method of treatment of a demyelinating disease selected from multiple sclerosis, schizophrenia, depression, Alzheimer's disease, Alzheimer-like dementia, Parkinson's disease and Huntington's chorea.

6. A compound according to claims 2-3 for use in a method of treatment of cardiac or renal ischaemia.

7. A compound according to claims 2-3 for use in a method of treatment of cerebral ischaemic injury.

8. A compound of formula (I), (II), (III), (IV) or (V), as defined in claim 1, for diagnostic use.

9. A pharmaceutical composition containing a compound of formula (I), (II), (III), (IV) or (V), as defined in claim 1, in admixture with a pharmaceutically acceptable carrier or excipient.

## Patentansprüche

1. Verbindung, ausgewählt aus der Gruppe, bestehend aus:
2-[[5-(2-Methoxyphenyl)-4-(4-methoxyphenyl)-4H-1,2,4-triazol-3-yl]thio]-N-phenylpropanamid der Formel (I): 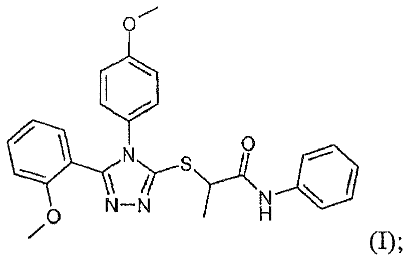
6-Methoxy-3-[[1-(2-methoxyethyl)-1H-tetrazol-5-yl][(2-phenylethyl)-(phenylmethyl)amino]methyl]-2(1H)-chinolinon der Formel (II): 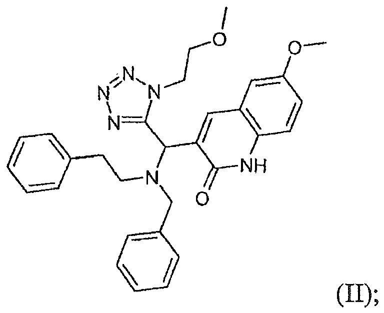
4-Amino-N5-(2-furanylmethyl)-N5-[1-(4-hydroxyphenyl)-2-[[(4-methoxyphenyl)methyl]amino]-2-oxoethyl]-3,5-isothiazol-dicarboxamid der Formel (III): 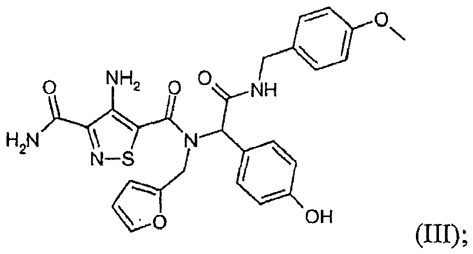
N-[1-(4-Fluorphenyl)-2-oxo-2-[[(tetrahydro-2-furanyl)methyl]amino]ethyl]-N-(4-methoxyphenyl)-1H-benzotriazol-1-acetamid der Formel (IV): 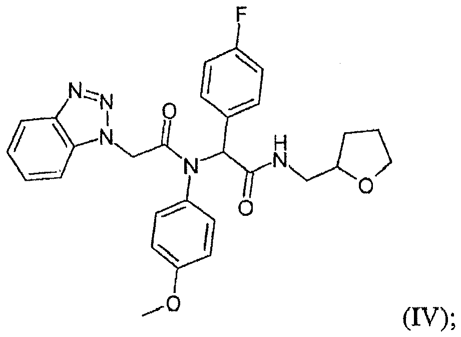
4-[3-[(2-Chlorphenyl)methyl]-6,7-dihydro-7-oxo-3H-1,2,3-triazol[4,5-d]-pyrimidin-5-yl]-N-[4-(trifluormethyl)phenyl]-1-piperidincarboxamid der Formel (V): 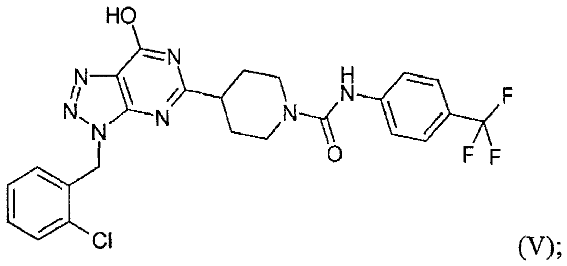
oder ein Salz, Isomer, einzelnes Enantiomer, Racemat oder Tautomer davon zur Verwendung als Therapeutikum.

2. Verbindung der Formel (I), (II), (III), (IV) oder (V), wie in Anspruch 1 definiert, zur Verwendung in einem Verfahren zur Behandlung von Krankheiten oder Dysfunktionen, die GPR17-Aktivierung involvieren.

3. Verbindung gemäß Anspruch 2 zur Verwendung in einem Verfahren zur Behandlung von akuten oder chronischen neurodegenerativen, inflammatorischen, Immunsystem-, kardiovaskulären, Nieren- und neoplastischen Krankheiten oder Dysfunktionen.

4. Verbindung gemäß den Ansprüchen 2 bis 3 zur Verwendung als Neuroprotektivum oder Mittel zur Wiederherstellung bei Ischämie und zerebralem Trauma.

5. Verbindung gemäß den Ansprüchen 2 bis 3 zur Verwendung in einem Verfahren zur Behandlung einer Demyelinationskrankheit, ausgewählt aus multipler Sklerose, Schizophrenie, Depression, Alzheimerscher Erkrankung, Alzheimer-artiger Demenz, Parkin-sonscher Erkrankung und Huntington-Chorea.

6. Verbindung gemäß den Ansprüchen 2 bis 3 zur Verwendung in einem Verfahren zur Behandlung von kardialer oder renaler Ischämie.

7. Verbindung gemäß den Ansprüchen 2 bis 3 zur Verwendung in einem Verfahren zur Behandlung von zerebraler ischämischer Verletzung.

8. Verbindung der Formel (I), (II), (III), (IV) oder (V), wie in Anspruch 1 definiert, zur diagnostischen Verwendung.

9. Pharmazeutische Zusammensetzung, die eine Verbindung der Formel (I), (II), (III), (IV) oder (V), wie in Anspruch 1 definiert, im Gemisch mit einem pharmazeutisch annehmbaren Träger oder Exzipiens enthält.

## Revendications

1. Composé choisi parmi le groupe constitué par :
le 2-[[5-(2-méthoxyphényl)-4-(4-méthoxyphényl)-4H-1,2,4-triazol-3-yl]thio]-N-phénylpropanamide répondant à la formule (I) : 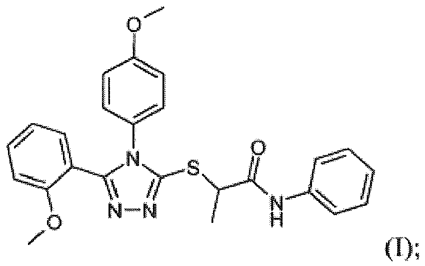
la 6-méthoxy-3[[1-(2-méthoxyéthyl)-1H-tétrazol-5-yl][(2-phényléthyl)-(phénylméthyl)amino]méthyl]-2(1 H)-quinolinone répondant à la formule (II) : 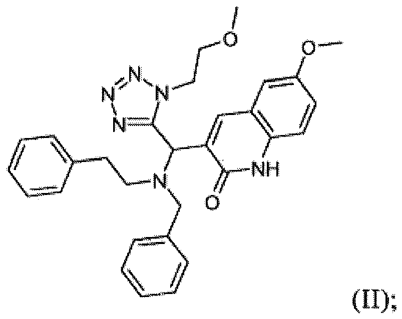
le 4-amino-N5-(2-furanylméthyl)-N5-[1-(4-hydroxyphényl)-2-[[(4-méthoxyphényl)méthyl]amino-2-oxoéthyl]-3,5-isothiazoledicarboxamide répondant à la formule (III) : 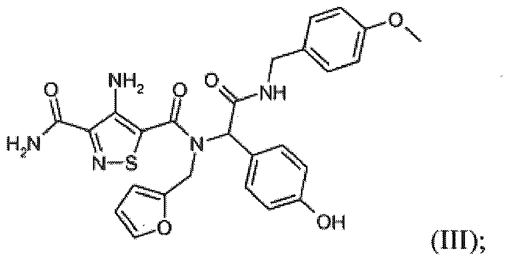
le N-[1-[4-fluorophényl)-2-oxo-2-[[(tétrahydro-2-furanyl)méthyl]amino]éthyl]-N-(4-méthoxyphényl)-1H-benzotriazol-1-acétamide répondant à la formule (IV) : 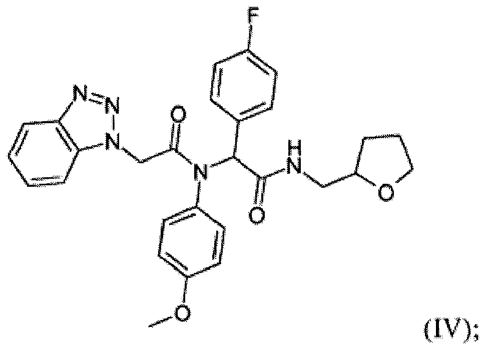
le 4-[3-[(2-chlorophényl)méthyl]-6,7-dihydro-7-oxo-3H-1,2,3-triazol[4,5-d]-pyrimidin-5-yl]-N-[4-(trifluorométhyl)phényl]-1-pipéridinecarboxamide répondant à la formule (V) : 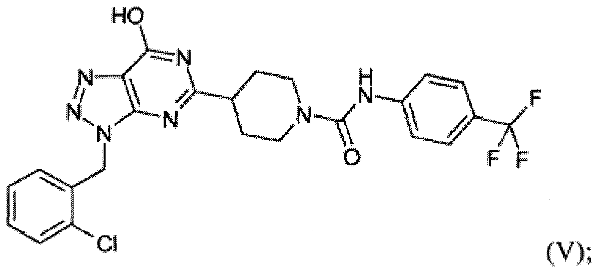
ou un de ses sels, un de ses isomères, un de ses énantiomères uniques, un de ses racémates ou un de ses tautomères, pour son utilisation comme agent thérapeutique.

2. Composé répondant à la formule (I), (II), (III), (IV) ou (V), tel que défini à la revendication 1, pour son utilisation dans un procédé de traitement de maladies ou de dysfonctionnements impliquant une activation du récepteur GPR17.

3. Composé selon la revendication 2, pour son utilisation dans un procédé de traitement de maladies ou de dysfonctionnements aigus ou chroniques de type neurodégénératif, inflammatoire, du système immunitaire, cardio-vasculaire, de type rénal et néoplasique.

4. Composé selon les revendications 2 à 3, pour son utilisation comme agent neuroprotecteur ou comme agent pour réparer une ischémie et un traumatisme cérébral.

5. Composé selon les revendications 2 à 3, pour son utilisation dans un procédé de traitement d'une maladie démyélinisante choisie parmi la sclérose en plaques, la schizophrénie, la dépression, la maladie d'Alzheimer, la démence de type Alzheimer, la maladie de Parkinson et la chorée de Huntington.

6. Composé selon les revendications 2 à 3, pour son utilisation dans un procédé de traitement d'une ischémie cardiaque ou rénale.

7. Composé selon les revendications 2 à 3, pour son utilisation dans un procédé de traitement d'une lésion ischémique cérébrale.

8. Composé répondant à la formule (I), (II), (III), (IV) ou (V), tel que défini à la revendication 1, pour une utilisation diagnostique.

9. Composition pharmaceutique contenant un composé répondant à la formule (I), (II), (III), (IV) ou (V), tel que défini à la revendication 1, en mélange avec un support ou un excipient pharmaceutiquement acceptable.
